# EUROPÄISCHE PATENTSCHRIFT

(11) **EP 2 238 426 B1**
(45) Veröffentlichungstag und Bekanntmachung des Hinweises auf die Patenterteilung: **20.02.2013**
(21) Anmeldenummer: 08871502.4
(22) Anmeldetag: 10.12.2008
(51) Int. Cl.: G01N 1/28, C12M 1/26, G02B 21/32

(54) **VERFAHREN UND VORRICHTUNG ZUM ÜBERTRAGEN EINER MIKROSKOPISCHEN, ISOLIERTEN PROBE, MIKRODISSEKTIONSSYSTEM MIT EINER DERARTIGEN VORRICHTUNG SOWIE VERFAHREN ZUR HERSTELLUNG EINES NANOSAUGERS**
METHOD AND DEVICE FOR TRANSFERRING A MICROSCOPIC, INSULATED SAMPLE, MICRO-DISSECTION SYSTEM COMPRISING SUCH A DEVICE AND METHOD FOR THE PRODUCTION OF A NANO-VACUUM
PROCÉDÉ ET DISPOSITIF POUR TRANSFÉRER UN ÉCHANTILLON MICROSCOPIQUE ISOLÉ, SYSTÈME DE MICRODISSECTION DOTÉ D'UN TEL DISPOSITIF, ET PROCÉDÉ DE FABRICATION D'UN NANO-ASPIRATEUR

(30) Priorität: 25.01.2008 EP 08150662
(43) Veröffentlichungstag der Anmeldung: 13.10.2010
(73) Patentinhaber: Helmholtz Zentrum München Deutsches Forschungszentrum für Gesundheit und Umwelt (GmbH), 85764 Neuherberg (DE); Ludwig-Maximilians-Universität München, 80539 München (DE)
(72) Erfinder: THALHAMMER, Stefan, 80639 München (DE); HÖHN, Norbert, 64295 Düsseldorf (DE); ZINK, Albert, 80538 München (DE); HECKL, Wolfgang, 80939 München (DE)
(74) Vertreter: Isarpatent
(86) Internationale Anmeldenummer: PCT/EP2008/067208
(87) Internationale Veröffentlichungsnummer: WO 2009/092495

(56) Entgegenhaltungen:
- EP-A- 0 539 888
- WO-A-98/03628
- WO-A-98/44972
- WO-A-2007/054161
- SCHINDLER M: "A cut above the rest" THE BIOCHEMIST, [Online] Oktober 2002 (2002-10), Seiten 25-28, XP002485626 Gefunden im Internet: URL:http://www.biochemist.org/bio/02405/00 25/024050025.pdf> [gefunden am 2008-06-25]
- STARK ROBERT W ET AL: "Combined nanomanipulation by atomic force microscopy and UV-laser ablation for chromosomal dissection." EUROPEAN BIOPHYSICS JOURNAL : EBJ MAR 2003, Bd. 32, Nr. 1, März 2003 (2003-03), Seiten 33-39, XP002485627 ISSN: 0175-7571

## Beschreibung

Die vorliegende Erfindung bezieht sich auf ein Verfahren und eine Vorrichtung zum Übertragen einer mikroskopischen, isolierten Probe, insbesondere eines membrangestützten Mikrodissektates, von einem Objekttisch zu einer Analyseeinrichtung, auf ein Mikrodissektionssystem, welches eine derartige Vorrichtung umfasst, sowie auf ein Verfahren zur Herstellung eines Nanosaugers für eine derartige Vorrichtung.

Obwohl auf beliebige mikroskopische, isolierte Proben anwendbar, werden die vorliegende Erfindung sowie die ihr zugrundeliegende Problematik in Bezug auf membrangestützte Mikrodissektate, insbesondere mittels der Laser-gestützten Mikrodissektion isolierte Mikrodissektate, näher erläutert.

Die Laser-gestützte Mikrodissektion ermöglicht es, kleinste Bereiche aus einem Gewebeschnitt bis hin zu einzelnen Zellen gezielt zu isolieren. Darüber hinaus kann mit der kontakt-freien Lasermanipulation die Mikrodissektion von einzelnen Zellkompartimenten, z.B. Chromosomen und Chromosomenfragmenten gewährleistet werden. Durch eine Kombination PCR, Klonierungstechniken und Laser- Mikrodissektion ist es möglich, Region-spezifische chromosomale Proben für die molekulare Zytogenetik zu entwickeln. Allerdings werden bei diesen Ansätzen nach der Laser- Mikrodissektion die Fragmente mit einer möglichst dünn gezogenen Glasnadel, vergleichbar mit der Glasnadel-Mikrodissektion, aufgesammelt. Eine Weiterentwicklung in der Laser-Mikrodissektion erfolgte in der Einführung der Laser-Capture-Mikrodissektion. Bei diesem Verfahren wird eine Kunststoffmembran auf das zu untersuchende Gewebe aufgebracht. Die zu isolierenden Zellen werden durch lokales Anschmelzen des Films thermisch aus dem Gewebe entfernt. Dieses Verfahren eignet sich besonders zur Isolierung von Zellgruppen bis zu einer Größe von ca. 30 µm.

Einen methodisch unterschiedlichen Ansatz zur Isolierung von Einzelzellen und Zellgruppen stellt das Prinzip der Laserdruck-Isolierung dar. Bei dieser Technik wird ein gepulster UV-Laser mit Leistungsspitzen von einigen Watt von unten auf die zu isolierende Geweberegion gerichtet. Das Gewebe ist im Gegensatz zur Laser-Capture-Mikrodissektion auf einer ultradünnen Trägermembran, welche eine Dicke von in etwa 1 bis 6 µm aufweist, aufgebracht. Mit dem UV-Laser können Zellen oder Zellgruppen gezielt isoliert werden, die in einem weiteren Arbeitsschritt mit Hilfe des Lichtdrucks auf eine Sammelvorrichtung nach oben isoliert werden, um sie für weitere biochemische Verfahren zur Verfügung zu stellen. Mit dieser Methode wurde die Isolierung von Chromosomen zur Entwicklung Chromosomen-spezifischer Paint-Proben vorgestellt.

Bei den oben genannten, bisher bekannten Ansätzen hat sich jedoch die Tatsache als nachteilig herausgestellt, dass keine definierte Übertragung der isolierten Probe gewährleistet ist und diese Ansätze eine Beschädigung beziehungsweise eine Verunreinigung der zu übertragenden Probe nachteilig hervorrufen können. Des Weiteren ist bei den oben genannten Ansätzen eine optische Kontrolle des gesamten Übertragungsvorgangs nur sehr umständlich, falls überhaupt, möglich. Ferner ist auch ein definiertes Aufnehmen und Ablegen der Probe aufgrund der verwendeten Übertragungsmittel nicht gewährleistet.

Beispielsweise treten Schädigungen von Gewebepartikeln, Zellen und Zellbestandteilen, Proteinen und kinetischem Material durch das Herauskatapultieren bei den oben genannten Ansätzen auf. Bei der Glasnadel-Mikrodissektion erfolgt nachteilig ein Abkratzen der Probe, so dass Beschädigungen der Probe in der Natur dieses Verfahrens liegen.

Aus dem Stand der Technik wird beispielweise Dokumente WO 98/03628, SCHINDLER M: "A cut above the rest", THE BIOCHEMIST, [Online] Oktober 2002, Seiten 25-28, im Internet: URL:http://www.biochemist.org/bio/02405/0025/024050025.pdf, WO 2007/054161 und EP 0 539 888 bekannt.

Folglich liegt der vorliegenden Erfindung in Anbetracht der oben genannten, bekannten Ansätze die Aufgabe zugrunde, die oben genannten Nachteile zu beseitigen und insbesondere eine Vorrichtung und ein Verfahren zum Übertragen einer mikroskopischen, isolierten Probe derart zu verbessern, dass eine schonendere und definiertere Übertragung der entsprechenden Probe von einem Objekttisch zu einer Analyseeinrichtung gewährleistet ist.

Diese Aufgabe wird erfindungsgemäß durch eine Vorrichtung mit den Merkmalen des Patentanspruchs 1, durch ein Mikrodissektionssystem mit den Merkmalen des Patentanspruchs 19, durch ein Verfahren mit den Merkmalen des Patentanspruchs 22 und durch ein Verfahren zur Herstellung eines Nanosaugers mit den Merkmalen des Patentanspruchs 26 gelöst.

Die der vorliegenden Erfindung zugrundeliegende Idee besteht darin, dass zur Übertragung einer mikroskopischen, isolierten Probe eine Ansaugeinrichtung verwendet wird, welche einen Nanosauger mit einem Saugrohr und einer Abschlussmembran und eine mit dem Nanosauger koppelbare Unter-/Überdruckeinheit zum Ansaugen beziehungsweise Abblasen der Probe auf die beziehungsweise von der Abschlussmembran aufweist; wobei eine Trägereinrichtung zum Tragen des Nanosaugers vorgesehen ist, welche mittels einer zugeordneten Positioniereinheit zum exakten Positionieren des Nanosaugers in einer Probenentnahmeposition zum Ansaugen der Probe auf die Abschlussmembran und in einer Probenabgabeposition zum Abblasen der Probe von der Abschlussmembran verstellbar ist.

Somit weist die vorliegende Erfindung gegenüber den bekannten Ansätzen den Vorteil auf, dass aufgrund des schonenden Ansaugens beziehungsweise Abblasens der Probe auf die beziehungsweise von der Abschlussmembran Beschädigungen der isolierten Probe verhindert werden und durch die Trägereinrichtung in Verbindung mit der zugeordneten Positioniereinheit eine exakte, definierte und kontrollierbare Übertragung der Probe auf einfache Weise gewährleistet wird.

In den Unteransprüchen finden sich vorteilhafte Ausgestaltungen und Verbesserungen der Vorrichtung und des Verfahrens zum Übertragen einer mikroskopischen, isolierten Probe, des Mikrodissektionssystems mit einer derartigen Vorrichtung sowie des Verfahrens zum Herstellen eines Nanosaugers, welcher in einer derartigen Vorrichtung Verwendung findet.

Vorzugsweise wird eine horizontale Einstellung der Entnahmestelle entweder motorisch gesteuert über einen Joystick unter Beobachtung durch die Mikroskopiereinheit angefahren oder automatisch per digitaler Positionsübergabe realisiert. Ein mechanisch am Nanosauger befestigter Abstandsmesser beziehungsweise Autofocus ermöglicht das µm-genaue Absenken des Nanosaugers bis zu einem vordefinierten Saugniveau über der Entnahmestelle. Der Abstandsmesser misst kontinuierlich die Ist-Distanz zur Oberfläche und vergleicht diese mit einer vorgegebenen Soll-Distanz. Erreicht der sich absenkende Nanosauger die vorgegebene Soll-Distanz, wird der Näherungsvorgang beendet und der Saugvorgang eingeleitet. Der Unterdruck saugt die Probe an die Abschlussmembran an und hält sie dort fest.

Anschließend wird der Nanosauger mittels der Positioniereinheit an der Abgabeposition positioniert. Während des gesamten Positioniervorgangs bleibt vorzugsweise der Unterdruck bestehen. Erreicht der Nanosauger seine Abgabeposition, wird er mittels des Abstandsmessers auf eine vordefinierte SollPosition analog zum oben erläuterten Vorgang abgesenkt. An dieser Position wird ein Überdruck angelegt, welcher die angesaugte Probe in den Analysebereich abbläst.

Die Erfindung wird im Folgenden anhand von Ausführungsbeispielen unter Bezugnahme auf die beiliegenden Figuren der Zeichnung näher erläutert. Von den Figuren zeigen:
- Fig. 1: eine schematische Teildarstellung einer Übertragungsvorrichtung gemäß einem bevorzugten Ausführungsbeispiel der vorliegenden Erfindung, welche in ein Mikrodissektionssystem integriert ist;
- Fig. 2: eine vergrößerte Querschnittsdarstellung des Kopfendes des Nanosaugers gemäß einem bevorzugten Ausführungsbeispiel der vorliegenden Erfindung;
- Fig. 3: eine Draufsicht auf eine Trägerplatte des Nanosaugers gemäß einem bevorzugten Ausführungsbeispiel der vorliegenden Erfindung;
- Fig. 4: eine schematische Darstellung einer Abschlussmembran bestehend aus Trägerplatte und Abschlussgitter gemäß einem bevorzugten Ausführungsbeispiel der vorliegenden Erfindung; und
- Fig. 5: eine Querschnittsansicht entlang der Schnittlinie A-A aus Fig. 2.

In den Figuren der Zeichnung bezeichnen gleiche Bezugszeichen gleiche oder funktionsgleiche Komponenten, soweit nichts Gegenteiliges angegeben ist.

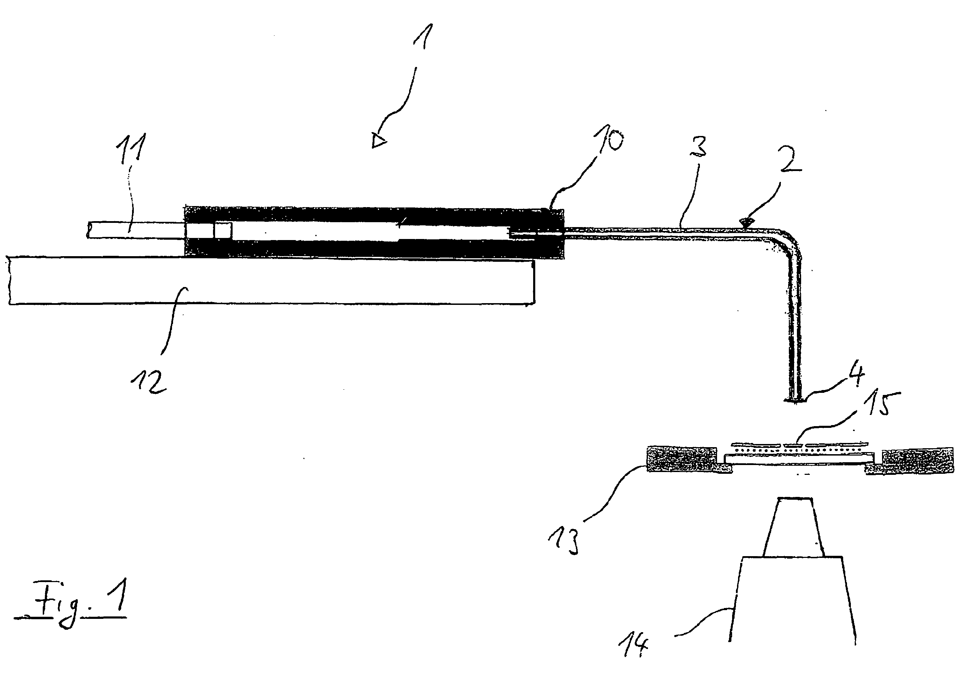

Fig. 1 illustriert eine schematische Teilansicht einer in ein Mikrodissektionssystem integrierten Übertragungsvorrichtung gemäß einem bevorzugten Ausführungsbeispiel der vorliegenden Erfindung zur Übertragung von isolierten Gewebestücken oder Zellen, im Folgenden als Probe 15 bezeichnet, insbesondere nach einer Laser-Mikrodissektion. Die Übertragungsvorrichtung ist vorzugsweise autonom ausgebildet und in bestehende Systeme integrierbar, beispielsweise an eine vorhandene Mikroskopiereinheit 14 des Mikrodissektionssystems fest anbringbar.

Die Übertragungsvorrichtung weist gemäß dem vorliegenden Ausführungsbeispiel eine Ansaugeinrichtung 1 auf, welche einem Ansaugen der Probe 15 von einem Objekttisch 13 in der in Fig. 1 dargestellten Probenentnahmeposition beziehungsweise einem Abblasen der Probe 15 in einer nicht dargestellten Probenabgabeposition dient. Der Objekttisch 13 kann beispielsweise sowohl als Mikroskopbühne einer zugeordneten Mikroskopiereinheit als auch als unabhängige Mikroskopbühne ausgebildet sein, in welcher ein Objektträger mit beispielsweise Trägermembran einspannbar ist. Dabei sei an dieser Stelle angemerkt, dass bei einer Laser-Mikrodissektion im Allgemeinen die Probe 15 auf einer Trägermembran angeordnet ist und zusammen mit dieser Membran durch die Übertragungsvorrichtung von dem Objekttisch 13 an eine nicht dargestellte Analyseeinrichtung transportiert wird.

Die Ansaugeinrichtung 1, wie in Fig. 1 schematisch illustriert, weist einen Nanosauger 2 auf, der beispielsweise aus einem Borsilikatglasröhrchen 3 und einer Abschlussmembran 4 besteht. Das Glasröhrchen 3 besitzt gemäß dem vorliegenden Ausführungsbeispiel eine L-Form, wie in Fig. 1 dargestellt, mit einem langen Schenkel und einem kurzen Schenkel. Der lange Schenkel dient einer luftdichten Verbindung mit einem Kopplungsröhrchen 10, welches weiter unten näher beschrieben wird.

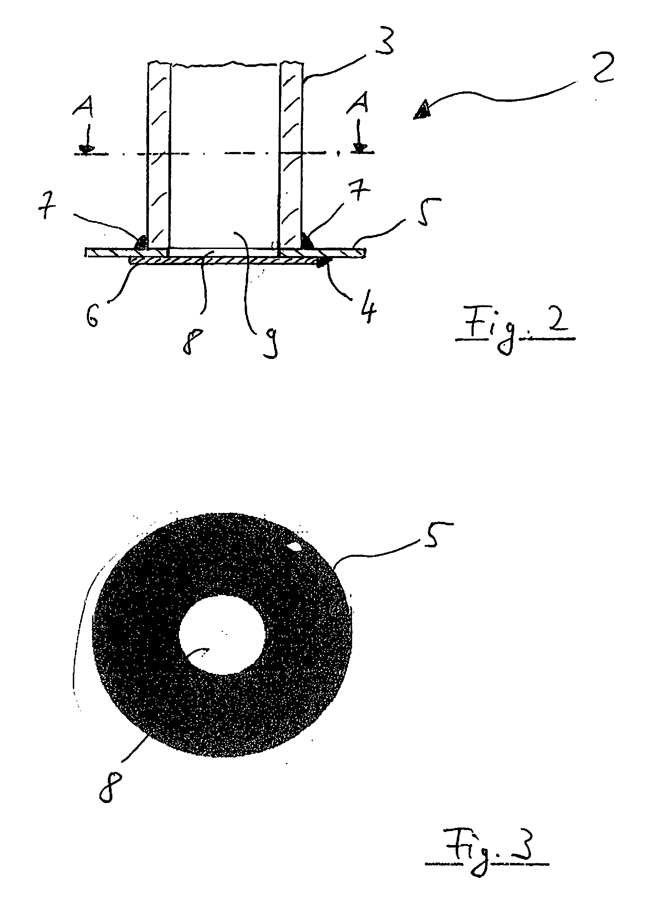

Der kurze Schenkel des Glasröhrchens 3 weist an seinem freien, stirnseitigen Ende eine Abschlussmembran 4 auf. Dieses Kopfende des Nanosaugers 2 ist in vergrößerter Darstellung in Fig. 2 in Querschnittsansicht illustriert. Das Glasröhrchen 3 weist beispielsweise einen Außendurchmesser zwischen in etwa 1,7 mm und 2,0 mm, einen Innendurchmesser zwischen in etwa 0,5 mm und 1,5 mm und eine Gesamtlänge, das heißt die Länge beider Schenkel zusammengenommen, von in etwa 50 mm bis 70 mm auf.

Des Weiteren ist das Saugrohr beziehungsweise Glasröhrchen 3 mit einer elektrisch-leitenden Schicht zumindest abschnittsweise, insbesondere im Endbereich des in Fig. 2 dargestellten Kopfbereiches, mit einer elektrisch-leitenden Goldschicht bedampft, beispielsweise mit einer Dicke von in etwa 60 nm.

Wie ferner in Fig. 2 ersichtlich ist, besteht die Abschlussmembran 4 aus einer Trägerplatte 5 und einem Abschlussgitter 6. Die Trägerplatte 5 ist in Fig. 3 gesondert und in vergrößerter Darstellung illustriert, wobei ersichtlich ist, dass die Trägerplatte 5 beispielhaft als Scheibe mit einem mittigen Loch 8 ausgebildet sein kann. Die Trägerplatte 5 besteht vorzugsweise aus einem elektrisch-leitfähigen Material, wie beispielsweise Kupfer oder dergleichen. Die Trägerplatte 5 ist unter Bezugnahme auf Fig. 2 vorzugsweise derart an der Stirnseite des Kopfabschnitts des Nanosaugers 2 befestigt, dass das Loch 8 mit der Öffnung 9 des Saugrohrs 3 fluchtet. Es ist aber auch vorstellbar, dass eine andere Ausgestaltung gewählt wird, jedoch sollte vorteilhaft die gesamte Saugleistung durch das Loch 8 weitergeführt werden.

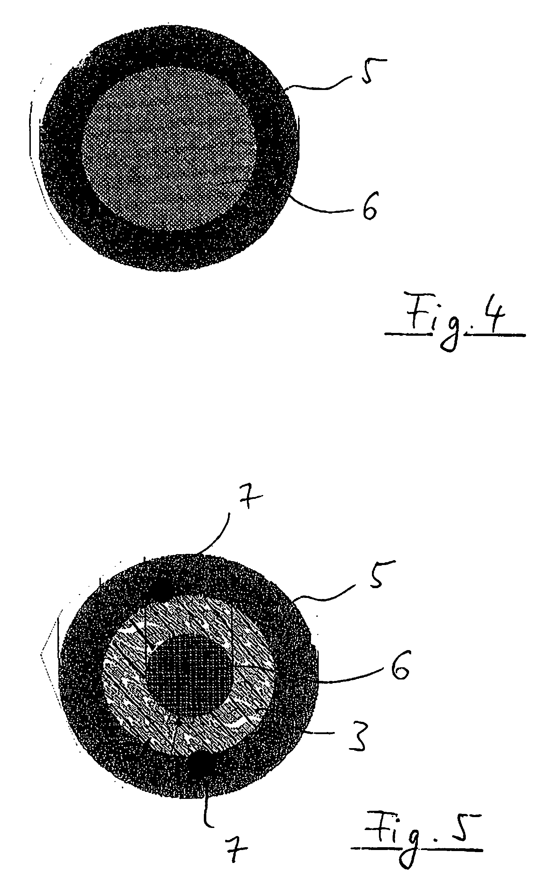

Die Abschlussmembran 4 weist ferner ein Abschlussgitter 6 auf, welches ebenfalls aus einem elektrisch-leitfähigen Material oder einem Material mit elektrisch leitender Oberfläche ausgebildet ist. In Fig. 4 ist eine Unteransicht der Abschlussmembran 4 bestehend aus Trägerplatte 5 und Abschlussgitter 6 dargestellt. Das Abschlussgitter 6 ist beispielsweise ebenfalls als runde Scheibe ausgebildet. Die Abmessungen des Nanosaugers, das heißt sowohl des Saugrohrs 3 als auch der Trägerplatte 5 und des Abschlussgitters 6 können in Abhängigkeit des jeweiligen Anwendungsfalls variiert werden. Insbesondere kann die Gitterstärke und die Porengröße des Abschlussgitters 6 an die jeweils zu übertragende Probe 15 in geeigneter Weise angepasst werden. Vorstellbar ist beispielsweise eine Gitterstärke von in etwa 20 µm und Poren mit einem Durchmesser zwischen in etwa 60 nm und 50 µm.

Das Abschlussgitter 6 ist vorzugsweise derart auf der Trägerplatte 5 aufgebracht, dass das Gitter das Loch 8 der Trägerplatte 5 und somit die Öffnung 9 des Saugrohrs 3 vollständig überdeckt, wie ebenfalls in Fig. 2 illustriert. Die gesamte Anordnung ist derart ausgebildet, dass die Saugluft direkt durch das Gitter 6 geleitet wird und nicht an irgendwelchen undichten Stellen zur Umgebung.

In Fig. 5 ist eine Querschnittsdarstellung entlang der Schnittlinie A-A aus Fig. 2 illustriert. Eine elektrostatische Aufladung des Nanosaugers 2 ist zum Schutze der Probe 15 zu verhindern. Wie in Fig. 5 in Verbindung mit Fig. 2 ersichtlich ist, sind für eine elektrische Verbindung zwischen der Trägerplatte 5 und der elektrisch-leitfähigen Beschichtung des Saugrohrs 3 elektrische Kontaktpunkte 7, beispielsweise in Form eines Leitsilbers oder dergleichen, vorgesehen. Dadurch sollen elektrostatische Aufladungen, die beispielsweise durch bestimmte Proben oder das Material der Abschlussmembran 4 hervorgerufen werden, vorteilhaft über das Saugrohr 3 bzw. einer dem Saugrohr 3 zugeordneten Erdungsleitung abgeleitet werden.

Zur Beschreibung weiterer Elemente der Übertragungsvorrichtung wird erneut Bezug genommen auf Fig. 1, wobei der Übersichtlichkeit halber die Übertragungsvorrichtung lediglich schematisch und ausschnittsweise dargestellt ist.

Beispielsweise weist die Ansaugeinrichtung 1, wie oben bereits angesprochen, ein Kopplungsröhrchen 10 auf, in welches das freie Ende des langen Schenkels des Saugrohrs 3 in luftdichter Weise in leicht austauschbarer Weise einsetzbar und arretierbar ist. Vorzugsweise weist das Kopplungsröhrchen einen Klips- oder Drehverschluss auf, mittels welchem der Nanosauger 2 von der Ansaugeinrichtung 1 zum Austauschen desselben lösbar verbindbar ist. Beispielsweise ist ein Drehverschluss mit einer entsprechenden Gummidichtung vorgesehen, welcher durch entsprechendes Drehen desselben das zugeordnete Ende des Saugrohrs 3 entweder in luftdichter Weise in dem Kopplungsröhrchen 10 aufnimmt oder für ein Entfernen des Saugrohrs 3 aus dem Kopplungsröhrchen 10 freigibt.

An dem gegenüberliegenden Ende des Kopplungsröhrchens 10 ist ein Anschluss 11 zum Anlegen einer zugeordneten, nicht dargestellten Unter-/Überdruckeinheit vorgesehen. Hier ist es ebenfalls vorstellbar, einen entsprechenden adaptiven Klips - oder Drehverschluss vorzusehen. Es ist auch vorstellbar, dass ein passgenauer Teflonschlauch über das entsprechende Ende des Kopplungsröhrchens 10 übergestülpt und mit Silikon zusätzlich versiegelt wird.

Die Unter-/Überdruckeinheit ist beispielsweise als pneumatische Pico-Pumpe ausgebildet, welche den jeweils gewünschten Unterdruck zum Ansaugen der Probe 15 auf das Abschlussgitter 6 beziehungsweise den jeweils gewünschten Überdruck zum Abblasen der Probe 15 von dem Abschlussgitter 6 bereitstellt. Beispielsweise wird eine empfindliche PLI-100 Drucksteuereinheit verwendet, welche eine genaue Einstellung sowohl eines niedrigen Drucks von beispielsweise 0 bis 0,75 kPa als auch eines hohen Druckes, von beispielsweise 413 kPa, für das Probenansaugen beziehungsweise -abblasen ermöglicht. Die Saugleistung beziehungsweise die Stärke des Abblasimpulses der Unter-/Überdruckeinheit wird entsprechend des jeweiligen Anwendungsfalls vorzugsweise vorab bestimmt und entsprechend eingestellt. Eine Regelbarkeit der Überdruckdauer von wenigen ms ist ebenfalls vorteilhaft, um die aufgenommene Probe sicher in einen vorgesehenen Flüssigkeitstropfen auf der Analyseeinrichtung abgeben zu können ohne diesen Tropfen zu zerstäuben.

Die Übertragungsvorrichtung umfasst des Weiteren, wie in Fig. 1 dargestellt ist, eine Trägereinrichtung 12, auf welchem zumindest das Kopplungsröhrchen 10 und somit der Nanosauger 2 der Ansaugeinrichtung 1 fest angebracht sind. Die Trägereinrichtung 12 ist mit einer zugeordneten, nicht dargestellten Positioniereinheit gekoppelt. Vorzugsweise sind zur Verstellung der Trägereinrichtung 12, wie oben bereits erwähnt, vier Freiheitsgrade der Positioniereinrichtung vorgesehen, drei translatorische Bewegungen (x-, y-, z-Richtung), sowie eine rotatorische Bewegung zum Verschwenken der Trägereinrichtung 12 in horizontaler Ebene. Beispielsweise wird ein Mikrometer-Schrittmotor verwendet, welcher die Trägereinrichtung 12 und somit den Nanosauger 2 mit Genauigkeit im µm-Bereich positionieren kann. Im Falle einer Anbringung der Übertragungsvorrichtung an einer zugeordneten Mikroskopiereinheit 14 kann dadurch lediglich der Objekttisch entsprechend verstellt werden, wobei bei einer Verschwenkung der Trägereinrichtung 12 eine exakte Positionierung des Nanosaugers über dem Linsensystem bzw. Lasersystem der Mikroskopiereinheit 14 gewährleistet ist, da aufgrund der festen Anbringung die Verstellung der Trägereinrichtung 12 in dem Bezugssystem der Mikroskopiereinheit 14 erfolgt. Dadurch wird eine maximale Genauigkeit und ein schnelles Aufnehmen der jeweiligen Proben, auch noch so kleiner Proben, gewährleistet. Die gesamte Verstellbewegung wird mittels einer entsprechenden Software und Steuer-/Regeleinheit ausgeführt.

Des Weiteren kann die Übertragungsvorrichtung bei Bedarf weitere Komponenten aufweisen, die der Übersichtlichkeit halber in den Figuren nicht dargestellt sind. Beispielsweise ist eine Visualisierungseinrichtung vorgesehen, welche einer optischen Kontrolle und Anzeige des Übertragungsvorgangs dient.

Die Visualisierungseinrichtung kann beispielsweise als CCD-Kamera mit einer flexiblen A.P.I.-Schnittstelle ausgebildet sein. Ferner ist ein Abstandsmesser, beispielsweise ein geeigneter Lasersensor vorgesehen, welcher online den Abstand zwischen der Abschlussmembran 4 und der aufzunehmenden Probe 15 beziehungsweise den Abstand zu einer Analyseeinrichtung misst, auf welche die aufgenommene Probe 15 abzulegen ist. Beispielsweise kann als Lasersensor eine LTA-02 Lasersensor-Autofocuseinheit für eine automatische Abstandsregelung verwendet werden, welche einen Sollabstand von zum Beispiel 100 µm zwischen den relevanten Oberflächen automatisch einstellt. Vorteilhaft kann auch dieser Lasersensor mechanisch mittels des oben genannten µm-Schrittmotors verstellbar ausgebildet sein.

Die Auflistung oben genannter Bauteile ist nicht abschließend zu verstehen, wobei für einen Fachmann offensichtlich ist, welche zusätzlichen Bauteile er in dem jeweiligen Anwendungsfall mit in die Übertragungsvorrichtung integrieren kann. Des Weiteren ist es für einen Fachmann auch selbstverständlich, die oben genannten Bauteile über die gemeinsame Steuer-/Regeleinheit und entsprechender Software derart miteinander zu verknüpfen, dass eine benutzerfreundliche Übertragung der jeweiligen Probe 15 von dem Objekttisch 13 zu einer zugeordneten Analyseeinrichtung geliefert wird, beispielsweise mittels eines zugeordneten Joysticks und Anzeige auf einem entsprechenden Monitor.

Wie oben bereits angedeutet, kann es bevorzugt sein, die beschriebene Übertragungsvorrichtung in ein Mikrodissektionssystem zu integrieren. In diesem Fall ist es vorteilhaft, die Übertragungsvorrichtung, welche als autonomes System ausgebildet ist, fest an einer vorhandenen Mikroskopiereinheit 14 anzubringen, so dass ein gemeinsames Bezugssystem gegeben ist. Dadurch wird die Positionierung des Nanosaugers 2 über dem Linsen- und/oder Lasersystem der Mikroskopiereinheit 14 erheblich erleichtert, da beispielsweise lediglich mittels einer rotatorischen Schwenkbewegung der Trägereinrichtung 12 eine exakte Positionierung bewerkstelligt wird. In diesem Fall ist lediglich noch eine entsprechende Absenkung des Nanosaugers 2 mittels des zugeordneten Abstandsmessers sowie eine horizontale Verstellung des Objekttisches 13 und somit der Probe 15 zur Feinjustierung von Nöten.

Vorteilhaft ist hierbei, dass die Übertragungsvorrichtung prinzipiell unabhängig und somit autonom von den weiteren Analyseschritten funktioniert und somit modular in bestehende Systeme eingebaut werden kann. Damit eröffnet sich ein großes Potential hinsichtlich der wissenschaftlichen Verwendung, aber auch gerade für eine später mögliche wirtschaftliche Vermarktung dieser Technologie.

Im Folgenden wird ein bevorzugtes Ausführungsbeispiel für ein Verfahren zum Herstellen eines Nanosaugers 2 näher erläutert, welcher für eine exakte Übertragung einer Probe 15 ohne Beschädigung und Verunreinigung dieser eine tragende Rolle spielt.

Zunächst wird beispielsweise ein Glasröhrchen 3 aus Borsilikatglas mit einem Außendurchmesser zwischen in etwa 1,7 mm und 2,0 mm und einem Innendurchmesser zwischen in etwa 0,5 mm und 1,5 mm mittels eines entsprechenden Gerätes in eine L-Form derart gebracht, dass das Glasröhrchen 3 vorzugsweise einen längeren Schenkel und einen kürzeren Schenkel aufweist, wie in Fig. 1 ersichtlich ist. Das derart gebogene Glasröhrchen 3 wird anschließend in einem vorbestimmten Bereich, vorzugsweise in dem Kopfabschnitt des kürzeren Schenkels mit einer beispielsweise 60 nm starken Goldschicht bedampft. Diese Goldschicht bewirkt eine elektrische Leitfähigkeit der entsprechenden Oberfläche des Glasröhrchens 3, verändert jedoch die transparenten Eigenschaften des Glasröhrchens 3 nicht.

Anschließend wird der Rand des kürzeren Schenkels des Glasröhrchens 3 mit vorzugsweise einem UV-aushärtenden Klebstoff bestrichen. Nachfolgend wird das Glasröhrchen 3 exakt zentrisch über der Trägerplatte 5 platziert und soweit mikromechanisch abgesenkt, dass der klebstoffbenetzte Rand des Glasröhrchens 3 die Trägerplatte 5 berührt. Nachfolgend wird der UV-Kleber mit einer UV-Lampe ausgehärtet und es entsteht eine bleibende Verbindung zwischen der Trägerplatte 5 und dem Glasröhrchen 3.

Nachfolgend wird auf der freien Unterseite der Trägerplatte 5 eine beispielsweise 0,5 mm breite Klebstoffspur aus einem vorzugsweise UV-aushärtenden Klebstoff um das dort befindliche Loch 8 der Trägerplatte 5 aufgebracht. Anschließend wird das Glasröhrchen 3 mitsamt vorher aufgebrachter Trägerplatte 5 mikromechanisch auf das Abschlussgitter 6 abgesenkt, wobei nach einem Kontakt zwischen der Trägerplatte 5 und dem Abschlussgitter 6 der aufgebrachte Kleber wiederum mittels einer UV-Lampe ausgehärtet wird.

Abschließend wird eine elektrisch-leitende Verbindung zwischen der Trägerplatte 5 und der leitenden Oberfläche beziehungsweise der aufgedampften, elektrisch-leitfähigen Schicht des Glasröhrchens 3 hergestellt, was beispielsweise mittels zweier Tropfen Leitsilber an gegenüberliegenden Abschnitten bewerkstelligbar ist.

Vorzugsweise weist die Übertragungsvorrichtung einen Satz an unterschiedlichen Nanosaugern 2 auf, wobei die einzelnen Nanosauger 2 sich unterscheidende Abschlussmembrane 4 beziehungsweise Abschlussgitter 6 aufweisen. Dabei können sich die Abmessungen, das Material, die Porengröße und Porenanordnung sowie weitere Eigenschaften des Abschlussgitters 6 voneinander unterscheiden, so dass in Abhängigkeit der jeweils zu übertragenden Probe 15 ein entsprechend geeigneter Nanosauger 2 ausgewählt und mit dem Kopplungsröhrchen 10 zum Integrieren in die Ansaugeinrichtung und somit in die Übertragungsvorrichtung manuell luftdicht verbindbar ist. Folglich kann der Benutzer auf einfache Art und Weise in Abhängigkeit seiner jeweils zu übertragenden Probe 15 manuell den geeigneten Nanosauger 2 aus dem vorhandenen Satz auswählen und in die Übertragungsvorrichtung einbauen. Dies hat im Übrigen auch den Vorteil, dass etwaige beschädigte Nanosauger auf einfache Art und Weise ausgetauscht werden können.

Im Folgenden wird noch unter Bezugnahme auf insbesondere Fig. 1 ein Verfahren zum Übertragen einer Probe 15 von einem Objekttisch 13 zu einer Analyseeinrichtung gemäß einem bevorzugten Ausführungsbeispiel der vorliegenden Erfindung näher erläutert. Hierbei wird exemplarisch davon ausgegangen, dass die Übertragungsvorrichtung an einer Mikroskopiereinheit 14 fest montiert ist und eine Probe mittels einer Laser-Mikrodissektion durch ein zugeordnetes Mikrodissektionssystem isoliert wird.

Zunächst wird ein Probenkörper auf einer zugeordneten Glasplatte des Objekttisches 13 platziert und über ein Linsensystem beziehungsweise ein Lasersystem der Mikroskopiereinheit 14 positioniert. Anschließend wird mittels beispielsweise der Laser-Mikrodissektion ein Bereich des Probenkörpers ausgeschnitten zum Isolieren der zu übertragenden Probe 15. Nachfolgend wird der Nanosauger 2 durch Verstellen der Trägereinrichtung 12 durch die Positioniereinheit in horizontaler Ebene über die Probe 15 beziehungsweise das Linsensystem der Mikroskopiereinheit 14 verschwenkt, und, falls notwendig, in seiner Höhe mittels des zugeordneten Abstandsmessers automatisch oder manuell eingestellt.

Nachfolgend wird durch Einstellen eines niedrigen Druckes an der Unter-/Überdruckeinheit, beispielsweise eines Druckes kleiner 0,75 kPa, die isolierte Probe 15 von dem Objekttisch 13 auf das Abschlussgitter 6 des Nanosaugers 2 gesaugt. Die Trägereinrichtung 12 wird anschließend unter Aufrechterhaltung des Ansaugdruckes von dem Objekttisch 13 zu der zugeordneten Analyseeinrichtung mittels der vorhandenen Positioniereinrichtung exakt verstellt.

Beispielsweise wird zur Analyse der zu übertragenden Probe 15 ein kleiner Flüssigkeitstropfen auf der Analyseeinrichtung vorgesehen, welcher der Aufnahme der Probe 15 dient. Anschließend wird die Trägereinrichtung 12 wiederum abgesenkt in einem vorbestimmten Abstand, beispielsweise 100 µm oberhalb der Oberfläche des Tropfens, wobei die Probe 15 durch Umschalten der Unter-/Überdruckeinheit auf einen kurzen Impuls mit einem höheren Druck, beispielsweise einem Druck von 413 kPa für 2 ms, auf den Tropfen in exakter und schonender Weise abgeblasen wird.

Dadurch ist es möglich, die isolierte Probe 15 für weiterführende Analysen in ein Reaktionsgefäß in exakter und schonender Weise zu überführen.

Der Nanosauger 2 wird, wie oben bereits erwähnt, beim Ansaugvorgang und beim Abblasvorgang vorzugsweise in einem in etwa 100 µm Abstand von der Probenoberfläche beziehungsweise zur Oberfläche des Tropfens geführt. Dies wird beispielsweise durch die Integration eines LTA-02 Lasersensors gewährleistet. Bei Annäherung des Nanosaugers 2 an die entsprechende Oberfläche misst der Lasersensor permanent den Abstand zur Oberfläche. Diese Differenz zwischen Soll- und Ist-Abstand wird an die Steuer-/Regeleinheit übermittelt und von dort wieder in die Bewegung des Nanosaugers 2 beziehungsweise der Trägereinrichtung 12 in Focusrichtung umgesetzt. Solange eine Differenz größer 0 existiert, wird die Bewegung fortgeführt. Entspricht der Soll-Abstand dem Ist-Abstand, stoppt die Focusbewegung des Nanosaugers und der Saugvorgang beziehungsweise der Abblasvorgang kann gestartet werden.

Obwohl die vorliegende Erfindung anhand bevorzugter Ausführungsbeispiele vorliegend beschrieben wurde, ist sie darauf nicht beschränkt, sondern auf vielfältige Weise modifizierbar. Insbesondere sind oben beispielhaft angegebene Abmessungen, Materialien und Eigenschaften der Komponeneten entsprechend der jeweiligen Anwendung modifizierbar. Entscheidend ist lediglich, dass eine Übertragung der isolierten Probe mittels einer entsprechenden Ansaugeinrichtung durchgeführt wird, welche mittels einer Trägereinrichtung in bestimmten Positionen exakt, insbesondere in horizontaler Verstellrichtung, positionierbar ist.

Somit weist die vorliegende Erfindung den Vorteil auf, dass Proben auf schonende, optisch überprüfbare und exakt reproduzierbare Art und Weise übertragbar sind, wobei das entsprechende System autonom und somit in bestehende Systeme integrierbar ausgebildet ist.

### Bezugszeichenliste

- 1: Ansaugeinrichtung
- 2: Nanosauger
- 3: Saugrohr
- 4: Abschlussmembran
- 5: Trägerplatte
- 6: Abschlussgitter
- 7: Gleitverbindung
- 8: Loch
- 9: Öffnung
- 10: Kopplungsröhrchen
- 11: Anschluss für Unter-/Überdruckeinheit
- 12: Trägereinrichtung
- 13: Objekttisch
- 14: Mikroskopiereinheit
- 15: Probe

## Patentansprüche

1. Vorrichtung zum Übertragen einer mikroskopischen, isolierten Probe (15), insbesondere eines membrangestützten Mikrodissektates, von einem Objekttisch (13) zu einer Analyseeinrichtung, mit:
einer Ansaugeinrichtung (1), welche einen Nanosauger (2) mit einem Saugrohr (3) und einer Abschlussmembran (4) an dem freien Ende des Saugrohrs (3) und eine mit dem Nanosauger (2) koppelbare Unter-/Überdruckeinheit zum Ansaugen beziehungsweise Abblasen der Probe (15) auf die beziehungsweise von der Abschlussmembran (4) aufweist; und
einer Trägereinrichtung (12) zum Tragen des Nanosaugers (2), welche mittels einer zugeordneten Positioniereinheit zum exakten Positionieren des Nanosaugers (2) in einer Probenentnahmeposition zum Ansaugen der Probe (15) auf die Abschlussmembran (4) und in einer Probenabgabeposition zum Abblasen der Probe (15) von der Abschlussmembran (4) verstellbar ist.

2. Vorrichtung nach Anspruch 1,
**dadurch gekennzeichnet,**
**dass** der Nanosauger (2) mittels eines Kopplungsröhrchens (10) mit der Unter-/Überdruckeinheit luftdicht koppelbar ist.

3. Vorrichtung nach Anspruch 2,
**dadurch gekennzeichnet,**
**dass** das Kopplungsröhrchen (10) Anschlusseinrichtungen, beispielsweise Steck- oder Klipsverbindungen, für einen manuell bewerkstelligbaren, luftdichten Anschluss der Unter-/Überdruckeinheit und/oder eines ausgewählten Nanosaugers (2) nach dem Baukastenprinzip derart aufweist, dass zumindest unterschiedliche Nanosauger (2) über das Kopplungsröhrchen (10) mit der Unter-/Überdruckeinheit auf einfache Weise manuell, luftdicht koppelbar sind.

4. Vorrichtung nach wenigstens einem der vorhergehenden Ansprüche,
**dadurch gekennzeichnet,**
**dass** das Saugrohr (3) eine in etwa L-förmige Ausgestaltung mit einer Kopplungsröhrchen-Anschlussseite und einer Abschlussmembran-Anschlussseite aufweist.

5. Vorrichtung nach wenigstens einem der vorhergehenden Ansprüche,
**dadurch gekennzeichnet,**
**dass** das Saugrohr (3) mit einer elektrisch-leitfähigen Schicht zumindest abschnittsweise beschichtet ist, insbesondere im Bereich der Abschlussmembran-Anschlussseite, beispielsweise mit einer Goldschicht mit einer Dicke von in etwa 60 nm.

6. Vorrichtung nach wenigstens einem der vorhergehenden Ansprüche,
**dadurch gekennzeichnet,**
**dass** die Abschlussmembran (4) eine scheibenförmige Trägerplatte (5), welche insbesondere aus einem elektrisch-leitfähigen Material, beispielsweise aus Kupfer oder dergleichen, ausgebildet ist, mit einem vorzugsweise mittigen Loch (8) und ein Abschlussgitter (6) aufweist, welches insbesondere aus einem Material mit elektrisch-leitfähiger Oberfläche besteht, insbesondere eine Gitterstärke und eine Porengröße, welche an die Abmessung und Art der zu übertragenden Probe (15) angepasst sind, beispielsweise eine Gitterstärke von in etwa 20 µm und Poren mit einem Durchmesser zwischen in etwa 600 nm und 5 µm aufweist und insbesondere biologisch inert, antistatisch und/oder UVC-resistent ausgebildet ist, wobei die Trägerplatte (5) mit der einen Seite an der Abschlussmembran-Anschlussseite des Saugrohres (3) derart befestigt ist, dass das Loch (8) der Trägerplatte (5) mit der Öffnung (9) des Saugrohrs (3) in etwa fluchtet, wobei das Abschlussgitter (6) an der anderen, freien Seite der Trägerplatte (5) derart befestigt ist, dass das Abschlussgitter (6) die Öffnung (9) des Saugrohrs (3) sowie das Loch (8) der Trägerplatte (5) überdeckt, und wobei insbesondere eine elektrisch-leitfähige Verbindung zwischen der Trägerplatte (5) und der elektrisch-leitfähigen Schicht des Saugrohrs (3).zu Erdungszwecken vorgesehen ist, beispielsweise mittels eines Leitsilbers oder dergleichen.

7. Vorrichtung nach wenigstens einem der vorhergehenden Ansprüche,
**dadurch gekennzeichnet,**
**dass** die Positioniereinheit für eine Rotationsbewegung und/oder eine Translationsbewegung der Trägereinrichtung (12) ausgebildet ist, beispielsweise unter Verwendung eines Mikrometer-Schrittmotors.

8. Vorrichtung nach wenigstens einem der vorhergehenden Ansprüche,
**dadurch gekennzeichnet,**
**dass** die Vorrichtung ferner einen Abstandsmesser, beispielsweise einen Lasersensor oder dergleichen, zum Messen des Abstandes zwischen der Abschlussmembran (4) und der zu übertragenden Probe (15) beziehungsweise der relevanten Oberflächen aufweist.

9. Vorrichtung nach wenigstens einem der vorhergehenden Ansprüche,
**dadurch gekennzeichnet,**
**dass** die Vorrichtung eine Visualisierungseinrichtung zur optischen Anzeige und Kontrolle des Übertragungsvorgangs aufweist.

10. Vorrichtung nach wenigstens einem der vorhergehenden Ansprüche,
**dadurch gekennzeichnet,**
**dass** die Vorrichtung eine Steuer-/Regeleinheit mitsamt geeigneter Software aufweist, welche für einen Datenaustausch, Ansteuerung, Regelung usw. mit der Trägereinrichtung (12), der Positioniereinrichtung, der Unter-/Überdruckeinheit, dem Abstandsmesser und/oder der Visualisierungseinrichtung verbunden ist.

11. Vorrichtung nach wenigstens einem der vorhergehenden Ansprüche,
**dadurch gekennzeichnet,**
**dass** die Vorrichtung autonom ausgebildet und modular in bestehende Systeme, beispielsweise einem Mikrodissektionssystem, integrierbar ist.

12. Mikrodissektionssystem, welches eine Vorrichtung nach wenigstens einem der vorhergehenden Ansprüche umfasst.

13. Mikrodissektionssystem nach Anspruch 12,
**dadurch gekennzeichnet,**
**dass** das Mikrodissektionssystem eine Mikroskopiereinheit aufweist, an welcher die Vorrichtung fest anbringbar ist, wobei die Vorrichtung insbesondere derart fest an der Mikroskopiereinheit anbringbar ist, dass die Trägereinrichtung (12) bezogen auf das Bezugssystem der Mikroskopiereinheit definiert verstellbar ist.

14. Verfahren zum Übertragen einer mikroskopischen, isolierten Probe (15), insbesondere eines membrangestützten Mikrodissektates, von einem Objekttisch (13) zu einer Analyseeinrichtung, mit folgenden Verfahrensschritten:
exaktes Positionieren eines Nanosaugers (2), welcher mit einem Saugrohr (3) und einer Abschlussmembran (4) ausgebildet und auf einer Trägereinrichtung (12) fest angebracht wird, in einer Probenentnahmeposition durch Verstellen der Trägereinrichtung (12) mittels einer zugeordneten Positioniereinheit;
Ansaugen der Probe (15) auf die Abschlussmembran (4) mittels einer mit dem Nanosauger (2) gekoppelten Unter-/Überdruckeinheit;
Verstellen der Trägereinrichtung (12) zum exakten Positionieren des Nanosaugers (2) in einer Probenabgabeposition mittels der zugeordneten Positioniereinheit; und
Abblasen der Probe von der Abschlussmembran (4) mittels der Unter-/Überdruckeinheit.

15. Verfahren nach Anspruch 14,
**dadurch gekennzeichnet,**
**dass** mittels eines Abstandsmessers, beispielsweise eines Lasersensors, der Abstand zwischen der Abschlussmembran (4) und der zu übertragenden Probe (15) beziehungsweise der relevanten Oberflächen kontinuierlich gemessen und mit einem Sollwert verglichen wird, wobei mittels einer Steuer-/Regeleinheit das Höhenniveau der Trägereinrichtung (12) solange nachgeregelt wird, bis der Ist-Abstandswert dem Soll-Abstandswert entspricht.

16. Verfahren nach Anspruch 14 oder 15,
**dadurch gekennzeichnet,**
**dass** ein geeigneter Unterdruck durch die Unter-/Überdruckeinheit während des gesamten Übertragungsvorgangs aufrechterhalten wird.

17. Verfahren nach wenigstens einem der Ansprüche 14 bis 16,
**dadurch gekennzeichnet,**
**dass** zum Abblasen der Probe (15) ein kurzer Druckimpuls mittels der Unter-/Überdruckeinheit erzeugt wird.

18. Verfahren zur Herstellung eines Nanosaugers (2), welcher in einer Vorrichtung zum Übertragen einer mikroskopischen, isolierten Probe (15), insbesondere eines membrangestützten Mikrodissektates, von einem Objekttisch (13) zu einer Analyseeinrichtung Verwendung findet, mit folgenden Verfahrensschritten:
Bereitstellen eines Glasröhrchens (3);
Umformen des Glasröhrchens (3) in eine in etwa L-Form;
Zumindest abschnittsweise Bedampfen des Glasröhrchens (3) mit einer elektrisch-leitfähigen Schicht;
Anbringen einer scheibenförmigen Trägerplatte (5), welche ein Loch (8) aufweist, mit einer Seite auf einem freien Ende des Glasröhrchens (3) derart, dass das Loch (8) der Trägerplatte (5) in etwa mit der Öffnung (9) des Glasröhrchens (3) fluchtet; und
Anbringen eines Abschlussgitters (6) auf der anderen Seite der Trägerplatte (5) derart, dass das Abschlussgitter (6) die Öffnung (9) des Glasröhrchens (3) sowie das Loch (8) der Trägerplatte (5) überdeckt.

19. Verfahren nach Anspruch 18,
**dadurch gekennzeichnet,**
**dass** der Schritt des Anbringens der Trägerplatte (5) an dem freien Ende des Glasröhrchens (3) das exakt zentrisch Platzieren des Glasröhrchens (3) über der Trägerplatte (5) und das soweit mikromechanisch Absenken beinhaltet, dass der klebstoffbenetzte Rand des Glasröhrchens (3) die Trägerplatte (5) berührt.

20. Verfahren nach Anspruch 18 oder 19,
**dadurch gekennzeichnet,**
**dass** der Schritt des Anbringens des Abschlussgitters (6) auf der anderen Seite der Trägerplatte (5) das Auftragen eines UV-aushärtenden Klebstoffs, beispielsweise in Form einer 0,5 mm breiten Klebstoffspur, auf der anderen Seite der Trägerplatte (5) um das Loch (8) der Trägerplatte (5) herum beinhaltet, wobei der Schritt des Anbringens des Abschlussgitters (6) auf der anderen Seite der Trägerplatte (5) ferner insbesondere das mikromechanisch Absenken des Glasröhrchens (3) mitsamt angebrachter Trägerplatte (5) auf das Abschlussgitter (6) beinhaltet, wobei nach Kontakt zwischen Trägerplatte (5) und Abschlussgitter (6) der vorgesehene Klebstoff mittels einer UV-Lampe ausgehärtet wird.

## Claims

1. Device for transferring a microscopic, isolated sample (15), in particular a membrane-supported micro-dissected specimen, from an object table (13) to an analysis device, having:
- a suction device (1) which comprises a nano-suction apparatus (2) with a suction tube (3) and a terminal membrane (4) at the free end of the suction tube (3) and a vacuum/overpressure unit which can be coupled to the nano-suction apparatus (2) for sucking and blowing the sample (15) onto and from the terminal membrane (4) respectively; and
- a carrier device (12) for carrying the nano-suction apparatus (2), which carrier device can be adjusted by means of an associated positioning unit for positioning the nano-suction apparatus (2) exactly in a sample removal position for sucking the sample (15) onto the terminal membrane (4) and in a sample release position for blowing the sample (15) from the terminal membrane (4).

2. Device as claimed in claim 1, **characterised in that** the nano-suction apparatus (2) can be coupled in an airtight manner to the vacuum/overpressure unit by means of a coupling tube (10).

3. Device as claimed in claim 2, **characterised in that** the coupling tube (10) comprises connection devices, e.g. plug-in or clip connections, for the connection, which can be effected manually, of the vacuum/overpressure unit and/or a selected nano-suction apparatus (2) in an airtight manner in accordance with the modular design principle such that at least different nano-suction apparatuses (2) can be coupled in a simple manner manually, in an airtight fashion, to the vacuum/overpressure unit via the coupling tube (10).

4. Device as claimed in at least one of the preceding claims, **characterised in that** the suction tube (3) comprises an approximately L-shaped configuration having a coupling tube-connection side and a terminal membrane-connection side.

5. Device as claimed in at least one of the preceding claims, **characterised in that** at least portions of the suction tube (3) are coated with an electrically conductive layer, in particular in the region of the terminal membrane-connection side, e.g. with a gold layer having a thickness of approximately 60 nm.

6. Device as claimed in at least one of the preceding claims, **characterised in that** the terminal membrane (4) comprises a disk-shaped carrier plate (5) which is formed in particular from an electrically conductive material, e.g. copper or the like, having a preferably central hole (8), and a terminal mesh (6) which consists in particular of a material having an electrically conductive surface, in particular comprises a mesh thickness and a pore size which are adapted to the dimension and type of sample (15) to be transferred, e.g. comprises a mesh thickness of approximately 20 µm and pores with a diameter between approximately 600 nm and 5 µm and is formed in particular to be biologically inert, antistatic and/or UVC-resistant, wherein the carrier plate (5) is attached with one side to the terminal membrane-connection side of the suction tube (3) such that the hole (8) in the carrier plate (5) is approximately flush with the opening (9) in the suction tube (3), wherein the terminal mesh (6) is attached to the other free side of the carrier plate (5) such that the terminal mesh (6) covers the opening (9) in the suction tube (3) and the hole (8) in the carrier plate (5), and wherein in particular an electrically conductive connection is provided between the carrier plate (5) and the electrically conductive layer of the suction tube (3) for earthing purposes, e.g. by means of a conductive silver or the like.

7. Device as claimed in at least one of the preceding claims, **characterised in that** the positioning unit is designed for a rotational movement and/or a translational movement of the carrier device (12), e.g. using a micrometer step motor.

8. Device as claimed in at least one of the preceding claims, **characterised in that** the device further comprises a distance meter, e.g. a laser sensor or the like, for measuring the distance between the terminal membrane (4) and the sample (15) to be transferred or the relevant surfaces.

9. Device as claimed in at least one of the preceding claims, **characterised in that** the device comprises a visualisation device for the visual display and monitoring of the transfer procedure.

10. Device as claimed in at least one of the preceding claims, **characterised in that** the device comprises a control/regulating unit together with appropriate software which is connected to the carrier device (12), the positioning device, the vacuum/overpressure unit, the distance meter and/or the visualisation device for data exchange, control and regulating purposes etc.

11. Device as claimed in at least one of the preceding claims, **characterised in that** the device is designed to be autonomous and can be integrated in a modular fashion into existing systems, e.g. a micro-dissection system.

12. Micro-dissection system which comprises a device as claimed in at least one of the preceding claims.

13. Micro-dissection system as claimed in claim 12, **characterised in that** the micro-dissection system comprises a microscope unit, to which the device can be fixedly attached, wherein the device can be fixedly attached to the microscope unit in particular in such a manner that the carrier device (12) can be adjusted in a defined manner in relation to the reference system of the microscope unit.

14. Method of transferring a microscopic, isolated sample (15), in particular a membrane-supported micro-dissected specimen, from an object table (13) to an analysis device, comprising the following method steps:
- exact positioning of a nano-suction apparatus (2), which is designed with a suction tube (3) and a terminal membrane (4) and is fixedly attached to a carrier device (12), in a sample removal position by adjusting the carrier device (12) by means of an associated positioning unit;
- suction of the sample (15) onto the terminal membrane (4) by means of a vacuum/overpressure unit which is coupled to the nano-suction apparatus (2);
- adjustment of the carrier device (12) for exact positioning of the nano-suction apparatus (2) in a sample release position by means of the associated positioning unit; and
- blowing the sample from the terminal membrane (4) by means of the vacuum/overpressure unit.

15. Method as claimed in claim 14, **characterised in that** the distance between the terminal membrane (4) and the sample (15) to be transferred or the relevant surfaces is measured continuously by means of a distance meter, e.g. a laser sensor, and is compared with a desired value, wherein the height level of the carrier device (12) is readjusted by means of a control/regulating unit until the actual distance value corresponds to the desired distance value.

16. Method as claimed in claim 14 or 15, **characterised in that** the vacuum/overpressure unit serves to maintain an appropriate vacuum during the entire transfer procedure.

17. Method as claimed in at least one of claims 14 to 16, **characterised in that** a short pressure pulse is generated by means of the vacuum/overpressure unit in order to blow the sample (15).

18. Method of producing a nano-suction apparatus (2) which is used in a device for transferring a microscopic, isolated sample (15), in particular a membrane-supported micro-dissected specimen, from an object table (13) to an analysis device, comprising the following method steps:
- providing a glass tube (3);
- reshaping the glass tube (3) into an approximate L-shape;
- vapour-coating at least sections of the glass tube (3) with an electrically conductive layer;
- attaching a disk-shaped carrier plate (5) which comprises a hole (8), with one side on a free end of the glass tube (3) such that the hole (8) in the carrier plate (5) is approximately flush with the opening (9) in the glass tube (3); and
- attaching a terminal mesh (6) to the other side of the carrier plate (5) such that the terminal mesh (6) covers the opening (9) in the glass tube (3) and the hole (8) in the carrier plate (5).

19. Method as claimed in claim 18, **characterised in that** the step of attaching the carrier plate (5) to the free end of the glass tube (3) includes centring the glass tube (3) exactly over the carrier plate (5) and lowering it micromechanically until the adhesive-coated edge of the glass tube (3) contacts the carrier plate (5).

20. Method as claimed in claim 18 or 19, **characterised in that** the step of attaching the terminal mesh (6) to the other side of the carrier plate (5) includes applying a UV-hardening adhesive, e.g. in the form of a 0.5 mm wide adhesive track, on the other side of the carrier plate (5) around the hole (8) in the carrier plate (5), wherein the step of attaching the terminal mesh (6) to the other side of the carrier plate (5) further includes in particular lowering the glass tube (3) micromechanically together with the attached carrier plate (5) onto the terminal mesh (6), wherein the adhesive provided is hardened by means of a UV-lamp after contact between the carrier plate (5) and the terminal mesh (6).

## Revendications

1. Dispositif pour transférer un échantillon microscopique isolé (15), en particulier une partie prélevée par microdissection et soutenue par une membrane, d'un plateau porte-objet (13) vers un équipement d'analyse, comprenant :
- un équipement d'aspiration (1), qui présente un nano-aspirateur (2) doté d'un tube aspirant (3) et d'une membrane terminale (4) au niveau de l'extrémité libre du tube aspirant (3), et une unité de dépression/surpression pouvant être accouplée au nano-aspirateur (2) pour aspirer ou évacuer par soufflage l'échantillon (15) sur ou hors de de la membrane terminale (4) ; et
- un équipement porteur (12) pour porter le nano-aspirateur (2), équipement qui, au moyen d'une unité de positionnement associée, pour le positionnement exact du nano-aspirateur (2), peut être déplacé dans une position de prélèvement d'échantillon pour aspirer l'échantillon (15) sur la membrane terminale (4), et dans une position de délivrance d'échantillon pour évacuer par soufflage l'échantillon (15) de la membrane terminale (4).

2. Dispositif selon la revendication 1, **caractérisé par le fait que** le nano-aspirateur (2) peut être accouplé de manière étanche au moyen d'un petit tube d'accouplement (10) à l'unité de dépression/surpression.

3. Dispositif selon la revendication 2, **caractérisé par le fait que** le petit tube d'accouplement (10) présente des équipements de raccordement, par exemple des raccordements par emboîtement ou par clipsage, pour un raccordement étanche manuellement gérable de l'unité de dépression/surpression et/ou d'un nano-aspirateur (2) sélectionné selon le principe de système modulaire de telle sorte qu'au moins différents nano-aspirateurs (2) peuvent être accouplés manuellement de manière étanche par l'intermédiaire du petit tube d'accouplement (10) avec l'unité de dépression/surpression de manière simple.

4. Dispositif selon au moins l'une des revendications précédentes, **caractérisé par le fait que** le type aspirant (3) présente une conception en forme approximative de L avec une face de liaison du petit tube d'accouplement et une face de liaison de la membrane terminale.

5. Dispositif selon au moins l'une des revendications précédentes, **caractérisé par le fait que** le tuyau aspirant (3) est revêtu au moins partiellement d'une couche électro-conductrice, en particulier dans la zone de la face de liaison de la membrane terminale, par exemple d'une couche d'or ayant une épaisseur d'environ 60 nm.

6. Dispositif selon au moins l'une des revendications précédentes, **caractérisé par le fait que** la membrane terminale (4) présente une plaque porteuse (5) en forme de disque, formée en particulier d'un matériau électro-conducteur, par exemple en cuivre ou similaire, dotée préférentiellement d'un trou central (8) et d'une grille d'arrêt (6), qui est constituée en particulier d'un matériau doté d'une surface électro-conductrice, en particulier une épaisseur de grille et une taille de pores adaptée à la dimension et au type d'échantillon (15) devant être transféré, présente par exemple une épaisseur de grille d'environ 20 µm et des pores ayant un diamètre situé environ entre 600 nm et 5 µm et est formé en particulier de manière biologiquement inerte, antistatique et/ou résistante aux UVC, la plaque porteuse (5) avec un côté au niveau de la face de liaison de la membrane terminale du tube aspirant (3) étant fixée de telle sorte que le trou (8) de la plaque porteuse (5) est sensiblement aligné avec l'ouverture (9) du tube aspirant (3), la grille d'arrêt (6) au niveau de l'autre côté libre de la plaque porteuse (5) étant fixée de telle sorte que la grille d'arrêt (6) couvre l'ouverture (9) du tuyau aspirant (3) ainsi que le trou (8) de la plaque porteuse (5), et en particulier une liaison électro-conductrice étant prévue pour une mise à la terre entre la plaque porteuse (5) et la couche électro-conductrice du tuyau aspirant (3), par exemple à l'aide d'un argent conducteur ou similaire.

7. Dispositif selon au moins l'une des revendications précédentes, **caractérisé par le fait que** l'unité de positionnement est formée pour un mouvement de rotation et/ou un mouvement de translation de l'équipement porteur (12), par exemple à l'aide d'un moteur pas-à-pas micrométrique.

8. Dispositif selon au moins l'une des revendications précédentes, **caractérisé par le fait que** le dispositif présente en outre un écartomètre, par exemple un capteur laser ou similaire, destiné à mesurer la distance entre la membrane terminale (4) et l'échantillon (15) devant être transféré ou les surfaces pertinentes.

9. Dispositif selon au moins l'une des revendications précédentes, **caractérisé par le fait que** le dispositif présente un équipement de visualisation destiné à l'affichage et au contrôle visuel du processus de transfert.

10. Dispositif selon au moins l'une des revendications précédentes, **caractérisé par le fait que** le dispositif présente une unité de commande et de réglage comprenant un logiciel adapté, qui est reliée pour un échange de données, la commande, le réglage, etc. avec l'équipement porteur (12), l'équipement de positionnement, l'unité de dépression/surpression, l'écartomètre et/ou l'équipement de visualisation.

11. Dispositif selon au moins l'une des revendications précédentes, **caractérisé par le fait que** le dispositif est formé de manière autonome et est intégrable de manière modulaire dans des systèmes existants, par exemple dans un système de microdissection.

12. Microdissection, qui comprend un procédé selon au moins l'une des revendications précédentes.

13. Système de microdissection selon la revendication 12, **caractérisé par le fait que** le système de microdissection présente une unité de microscopie, sur laquelle le dispositif peut être fixé solidement, le dispositif pouvant être en particulier fixé solidement sur l'unité de microscopie de telle sorte que le dispositif porteur (12) est réglable de manière définie en référence au système de référence de l'unité de microscopie.

14. Procédé pour transférer un échantillon microscopique isolé (15), en particulier une partie prélevée par microdissection et soutenue par une membrane, d'un plateau porte-objet (13) vers un équipement d'analyse, comprenant les étapes de traitement suivantes :
- le positionnement exact d'un nano-aspirateur (2), qui est formé d'un tuyau aspirant (3) et d'une membrane terminale (4) et est fixé solidement sur un équipement porteur (12), dans une position de prélèvement d'échantillon par déplacement de l'équipement porteur (12) à l'aide d'une unité de positionnement associée ;
- l'aspiration de l'échantillon (15) sur la membrane terminale (4) à l'aide d'une unité de dépression/surpression couplée au nano-aspirateur (2) ;
- le déplacement de l'équipement porteur (12) au positionnement exact du nano-aspirateur (2) dans une position de délivrance de l'échantillon à l'aide de l'unité de positionnement associée ; et
- l'évacuation par soufflage de l'échantillon hors de la membrane terminale (4) à l'aide de l'unité de dépression/surpression.

15. Procédé selon la revendication 14, **caractérisé par le fait que** la distance entre la membrane terminale (4) et l'échantillon à transférer (15) ou les surfaces pertinentes est mesurée en continu et comparée avec une valeur de consigne à l'aide d'un écartomètre, par exemple d'un capteur laser, le niveau de hauteur de l'équipement porteur (12) étant réglé de nouveau à l'aide d'une unité de commande/réglage jusqu'à ce que la valeur de distance réelle corresponde à la valeur de distance de consigne.

16. Procédé selon la revendication 14 ou 15, **caractérisé par le fait qu'**une dépression adaptée est maintenue par l'unité de dépression/surpression pendant la totalité du processus de transfert.

17. Procédé selon au moins l'une des revendications 14 à 16, **caractérisé par le fait qu'**une brève impulsion de pression est produite à l'aide de l'unité de dépression/surpression pour évacuer l'échantillon (15) par soufflage.

18. Procédé de fabrication d'un nano-aspirateur (2), qui est utilisé pour un équipement d'analyse dans un dispositif destiné à transférer un échantillon microscopique isolé (15), en particulier une partie prélevée par microdissection et soutenue par une membrane, d'un plateau porte-objet (13) vers un équipement d'analyse, comprenant les étapes de traitement suivantes :
- préparation d'un petit tube en verre (3) ;
- façonnage du petit tube en verre (3) en forme de L ;
- métallisation au moins partielle du petit tube en verre (3) avec une couche électro-conductrice ;
- mise en place d'une plaque porteuse en forme de disque (5), qui présente un trou (8), avec un côté sur une extrémité libre du petit tube en verre (3) de telle sorte que le trou (8) de la plaque porteuse (5) est sensiblement aligné avec l'ouverture (9) du petit tube en verre (3) ; et
- mise en place d'une grille d'arrêt (6) sur l'autre côté de la plaque porteuse (5) de telle sorte que la grille d'arrêt (6) couvre l'ouverture (9) du petit tube en verre (3) ainsi que le trou (8) de la plaque porteuse (5).

19. Procédé selon la revendication 18, **caractérisé par le fait que** l'étape de mise en place de la plaque porteuse (5) à l'extrémité libre du petit tube en verre (3) comprend le placement exactement centré du petit tube en verre (3) au-dessus de la plaque porteuse (5) et l'abaissement micro-mécanique de telle sorte que le bord humecté de colle du petit tube en verre (3) touche la plaque porteuse (5).

20. Procédé selon la revendication 18 ou 19, **caractérisé par le fait que** l'étape de mise en place de la grille d'arrêt (6) sur l'autre côté de la plaque porteuse (5) comprend l'application d'une colle durcissant aux UV, par exemple sous la forme d'une marque de colle large de 0,5 mm, sur l'autre côté de la plaque porteuse (5) autour du trou (8) de la plaque porteuse (5), l'étape de mise en place de la grille d'arrêt (6) sur l'autre côté de la plaque porteuse (5) comportant en outre en particulier l'abaissement micro-mécanique du petite tube en verre (3) comprenant la plaque porteuse mise en place (5) sur la grille d'arrêt (6), la colle prévue étant durcie à l'aide d'une lampe à UV après le contact entre la plaque porteuse (5) et la grille d'arrêt (6).
